# EUROPEAN PATENT SPECIFICATION

(11) **EP 0 877 619 B1**
(45) Date of publication and mention of the grant of the patent: **19.06.2002**
(21) Application number: 97943121.0
(22) Date of filing: 21.10.1997
(51) Int. Cl.: A61K 35/78, A61K 7/48, A61P 17/00

(54) **USE OF GINKGO BILOBA EXTRACTS FOR THE PREPARATION OF PHARMACEUTICAL COMPOSITIONS**
VERWENDUNG VON GINKGO BILOBA EXTRAKTEN ZUR HERSTELLUNG VON PHARMAZEUTISCHEN ZUSAMMENSTELLUNGEN
UTILISATION D'EXTRAITS DE GINKGO BILOBA DANS LA PREPARATION DE COMPOSITIONS PHARMACEUTIQUES

(30) Priority: 22.10.1996 FR 9612822
(43) Date of publication of application: 18.11.1998
(73) Proprietor: Johnson & Johnson Consumer France, 92130 Issy les Moulineaux (FR)
(72) Inventor: CASTELLI, Dominique, F-75017 Paris (FR); FRITEAU, Laurence, F-94000 Créteil (FR); RIES, Gerd, D-40474 Düsseldorf (DE)
(74) Representative: Martin, Jean-Jacques
(86) International application number: IB9701319
(87) International publication number: WO9817295

(56) References cited:
- EP-A- 0 352 146
- PATENT ABSTRACTS OF JAPAN vol. 017, no. 158 (C-1041), 29 March 1993 & JP 04 321616 A (KOSE CORP), 11 November 1992,
- DATABASE WPI Section Ch, Week 8713 Derwent Publications Ltd., London, GB; Class B04, AN 87-089858 XP002035002 & JP 62 039 521 A (KURARAY CO LTD) , 20 February 1985

## Description

The present invention relates to the use, in the immunomodulation field, of extracts capable of being obtained from Ginkgo biloba. These extracts are useful in particular for treating, preventing or modulating the skin manifestations of immune disorders.

Ginkgo biloba is a dioecious tree which is native to the Far East and whose leaves are used for the preparation of medicinal products.

Ginkgo extracts have been proposed for improving the symptoms of intellectual deficiency in elderly subjects, or of intermittent claudication, obliterating arteriopathies and Raynaud's disease; it is also recommended in the case of retinal deficiency, where the components of the Ginkgolide group would then be more particularly involved in the desired activity.

It is know from EP 352 146 that extracts of Ginkgo biloba can be useful by the oral route to regulate certain enzymes, and in particular ribonucleases and also plays a role in normalising the rate of gamma-globuline in cases such as auto-immun diseases or viral diseases.

Unexpectedly, the applicant has now found that extracts obtained from Ginkgo biloba possess an immunomodulatory activity via the topical route. Accordingly, the subject of the present invention is the use of at least one active principle, capable of being obtained by extraction from Ginkgo biloba, for the preparation of a composition with immunomodulatory activity via the topical route.

In a particular mode the active principle is present in the composition in the form of an extract obtained from Ginkgo biloba leaves.

Immune reactions are regulated by a complex system and are involved in maintaining the general good condition of the body. They contribute to the defence against foreign particles, in articular pathogenic agents, or against the cells of the host which have undergone a neoplastic transformation.

This defence involves the recognition of the foreign nature of an antigen and the activation of cellular or hormonal mediators. It is dependent on genetic factors, through the genes of the major histocompatibility complex.

However, this mechanism can sometimes be undesirable when it is exerted too intensively, or against environmental antigens which are not intrinsically harmful; this is also the case during organ or tissue transplants. Moreover, certain dysfunctions lead to an erroneous recognition of the host cells, which are destroyed by the host's own immune system, and may cause a range of pathologies (such as disseminated lupus erythematosus, dermatomyositis, rheumatoid arthritis or certain types of fulminant hepatitis for example).

It has moreover been observed that in allergic subjects, there was a prevalence of people also having a so-called "sensitive" skin. In general, individuals are increasingly complaining of such a sensitive skin. Although their etiology is complex, it seems that factors of an allergic and inflammatory nature are often involved.

The notion of sensitive skins covers a range of manifestations comprising reactive skins and intolerant skins. The triggering factors may be environmental aggressions such as wind, pollution, temperature variations, excessively hard water or unsuitable hygiene, cosmetic or care products; these phenomena may also be associated with stress or emotions felt by the subject, some diets or the taking of medicinal products. There are, in addition, individual predisposing factors (especially neurological or hormonal factors) or familial predisposing factors which amplify these reactions.

In general, the subject feels a skin discomfort which may manifest itself by subjective and/or objective signs. The skin is easily subject to stabbing pain, itching or pricking; there may be sensations of heat, biting or burning. The skin may be subject to reddening or desguamation. Xerosis, seborrhoeic dermatitis, telangiectasia, vesicles or even oedema may be observed irregularly.

This condition may manifest itself at the level of the skin, the mucous membranes or the scalp. In the latter case, it may be associated with a dandruff condition and/or alopecia.

As indicated above, this condition often comprises an allergic component. In addition, it may progress into dermatological diseases of the immunoallergic type such as atopy, eczema or neurodermatitis.

The allergic conditions have been classified into four types, based on the nature of the immunological reaction, and they result from an immunological mechanism of inflammation.

It has now been found that Ginkgo extracts have an immunomodulatory activity. This can be demonstrated on mixed lymphoepidermal cultures.

In a particular mode of the use of at least one active principle obtained by extraction from Gingko biloba according to the present invention, the composition is intended for the treatment or for the prevention of intolerance and/or allergy reactions of the skin and of the mucous membranes.

Indeed, the skin, and more particularly the epidermis where the Langerhans' cells are located, plays a role in the body's immune defence. It is the determination of the function of the Langerhans' cells (LC) as antigen-presenting cells and the fact that the keratinocytes can generate factors involved in the immune response which have been at the origin of the notion of cutaneous inmune system. The peripheral position of the Langerhans' cells confers on them an essential role in taking charge of, preparing and presenting the antigen to the T lymphocytes. The presenting of the antigen involves the interaction between the class II molecules of the major histocompatibility complex (HLA-DR) expressed by the Langerhans' cells and the T receptor of the CD4+ lymphocytes. During the early phase of sensitization, the antigens are taken charge of, in the epidermis, by the Langerhans' cells and converted into immunogenic peptides. The antigenic presentation to the T lymphocytes occurs in the ganglia through the interaction between the complex HLA-DR-immunogenic peptides at the surface of the Langerhans' cells and the T receptor of the CD4+ lymphocytes.

MLECs constitute a model which makes it possible to determine the immunomodulatory properties of a substance capable of modifying the cutaneous immunological equilibrium by measuring the lymphocyte proliferation induced by the antigen-presenting allogenic epidermal cells, with or without treatment with the substance.

The anti-allergic activity is confirmed on keratinocytes and macrophage cells. Macrophages are particularly advantageous cells because they are cells resident at the level of the skin which regulate not only local inflammatory reactions but also immune responses through their capacity to present the antigen and to produce a large number of cytokines which regulate local immunity. These macrophages are also involved in communications with the circulatory system which can, where appropriate, mobilize various cell types (monocytes, neutrophils, eosinophils and T lymphocytes), thus increasing the nonspecific and specific defensive power of the tissue considered.

The activity of the active principle extracted from Ginkgo has thus been evaluated on cultures of human keratinocytes and macrophages stimulated or otherwise by IL-4 which induces the CD23 receptor at the surface of the cells, and then by IgE-containing immune complexes. This type of stimulation reveals allergic inflammation (IgE); it places the cells in the context of a pro-oxidizing response (generation of superoxide anion or NO free radicals, the measurement of which may be evaluated by measuring the production of nitrogenous derivatives) and an immunoinflammatory response (production of cytokines such as TNF-α).

In addition, this immunomodulatory activity is enhanced by the fact that active principles extracted from Ginkgo also have an activity on inflammation which is nonspecific and locally observed.

More particularly, the active principle(s) useful according to the invention are present in the composition in the form of an extract obtained from Ginkgo biloba. Various Ginkgo biloba extracts may be suitable for carrying out the invention; preferably, the terpene concentration in these extracts is less than about 7% w/w.

Indeed, Ginkgo biloba has a complex chemical composition which comprises aliphatic alcohols and hydrocarbons, steroids, amino acids, polyphenols such as luteolin, quercetol and more specific biflavones derived from amentaflavone (bilobetin, ginkgetin); it also contains ginkgolic acid and sesquiterpene-chain-containing anacardic derivatives, terpenes derived from limonene and terpenes with tert-butyl groups (bilobalides and Ginkgolides).

Extracts appropriate for carrying out the invention may have a content of terpene derivatives with a value of less than about 3% w/w of dry matter, especially of less than 1% w/w and advantageously of less than 0.5% w/w.

Preferably, chlorophyll, lipids. waxes and lectins are substantially removed from the extract used.

The extract also substantially lacks substances such as ginkgolic acid, biflavonoids or free aglycones.

The concentration of proanthocyanidins is preferably less than 5% w/w of the dry extract.

Extracts which can be used according to the invention preferably have a concentration of flavone heterosides greater than 24% w/w of dry matter, more preferably greater than 28% w/w; it is advantageously between about 29 and 35% w/w.

The flavone heterosides are compounds whose aglycone is a flavonol such as quercetin, kaempferol or isorhamnetin. These are mono-, tri- and especially diglucosides, of which the principal carbohydrate constituents are glucose and rhamnose. They are present in a substantial proportion in the form of coumarin esters of quercetin and of kaempferol glucorhamnoside.

A Ginkgo extract which is particularly suitable for carrying out the invention has, for a water content of less than 3% w/w, a concentration of flavone heterosides of 32 ± 3% w/w, a Ginkgolic acid concentration of less than 10 ppm, a proanthocyanidin concentration of less than 5% w/w and a terpene concentration of less than 0.5% w/w.

According to one of its features, the invention therefore relates to the use of at least one active principle, capable of being obtained from Ginkgo biloba, for the preparation of a composition which makes it possible to prevent, treat and/or modulate sensitive skin reactions as defined above.

According to another feature, the invention relates to the use of active principles extracted from Ginkgo, as defined above, for the preparation of compositions intended for the treatment or prevention of a condition chosen from the group comprising: atopy, vitiligo, psoriasis, erythema multiforme, neurodermatitis, xeroderma, rosacea, urticaria, pemphigus, lupus erythematosus, dermatitis, benign aestival photodermatosis and eczema.

The Ginkgo biloba extracts may, in accordance with the invention, be used by adapting their dosage, to modulate certain immune mechanisms.

They may thus be proposed for certain dermatological conditions associated with AIDS; for example, the current understanding of Kaposi's disease, which conventionally exhibits 4 clinical forms (and for which a virus of the herpes group is supposed to be the causative agent), is that of a hyperplasia consisting especially of inflammatory cells.

Preferably, in the compositions according to the invention, the Ginkgo biloba extract is present in the composition at a concentration of between 0.0001 and 10% w/w, advantageously between 0.01 and 2% w/w.

The extracts may be used as such in formulations, or precombined with phospholipids which enhance their bioavailability in vehicles of the liposome type, or Phytosome^{⊙}.

The compositions according to the invention will be formulated with dermatologically acceptable excipients known to persons skilled in the art.

The following examples are intended to illustrate the invention.

In these examples, reference will be made to the following figure:
- **Figure :**: Proliferation of lymphoepidermal cells in the presence or otherwise of various concentrations of a Ginkgo biloba extract.

### Example 1

### 1. Materials and Methods

### Products

The following products were used during this study: IL-4 is obtained from Immugenex (Los Angeles, CA) and is used at 10 ng/ml, the monoclonal IgE's are obtained from Stallergene (Fresnes, France) and the anti-IgE's are from Nordic (Tilburg, Holland).

### Culture of keratinocytes

The primary cultures of keratinocytes are obtained from neonatal prepuces and are maintained in proliferation ex vivo in a medium not containing calf serum. The confluent cultures of keratinocytes are trypsinized and transferred into 24-well plates in fresh medium at a cell density of 10⁵ cells/ml/well. The presence of the IgE receptor (CD23) at the surface of the cells is checked by immunolabelling.

### Culture of macrophages

The macrophage cells are obtained from the peripheral blood of normal (nonallergic) donors. The mononuclear cells are isolated on a ficoll gradient and the ring of lymphoid cells is recovered, washed three times and then the cells are cultured so as to cause the macrophage cells to adhere. These cells are recovered after adhering for 1 hour and are cultured (10⁶ cells/ml/well). Before stimulation by IL-4, the presence of the IgE receptor (CD23) at the surface of the cells is checked by immunolabelling.

### Cell activation

The cells are first activated by IL-4 so as to induce CD23 and are then cultured and stimulated by IgE-containing immune complexes for 3 to 5 days before recovering the various supernatants. The nitrogenous derivatives are assayed using Griess and the TNF level is measured using assay kits from Medgénix (Fleurus, Belgium).

### 2. Results

### Effect of a Ginkgo biloba extract on the keratinocytes and the macrophages stimulated by IL-4

Dry Ginkgo biloba leaves were subjected to continuous extraction with an acetone-water mixture under vacuum, and several steps of removal of solvent as well as of chlorophyll, lipids, waxes, lectins and of certain substances lead to an extract called hereinafter H37.

It exists in the form of a fine powder corresponding to the following specifications:
- residual solvents
   · ethanol < 3%
   · acetone < 0.1%
   · butanol < 0.1%
   · ethyl acetate < 0.1%
- sulphated ash < 1.5%
- water content < 3%
- proanthocyanidins < 5%
- terpenes < 0.5%
- ginkgolic acid < 10 ppm
- heavy metals < 20 ppm
- flavone heterosides 32 ± 3%

It is soluble at 6% (w/w) in PEG 400 and at 4% (w/v) in 90% ethanol.

The cells are stimulated for 48 h in the presence of IL-4 (10 ng/ml) so as to induce the receptor of low affinity for the IgE's (CD23) at their surface. At the end of this culture period, 30 to 80% of the keratinocytes and of the macrophages express CD23. The individual variations are in no case the reflection of a different allergic situation between these individuals. Whatever the situation, in this induction phase, the tested product does not modify this induction of CD23; indeed, a decrease of less than 5% cannot be observed (n = 8).

**Table 1:**

| Induction of the expression of CD23 by the various cells stimulated by IL-4 in the presence or otherwise of 10 mg/ml H37 | | |
|---|---|---|
| Cells | Medium | + IL-4 |
| Macrophages | < 5% | 45 ± 4 |
| + H37 | < 5% | 41 ± 2 |
| Keratinocytes | ND | 55 ± 7 |
| + H37 | ND | 51 ± 4 |
| * The cells are stimulated for 48 h in the presence or otherwise of 10 ng/ml of IL-4 and in the presence or in the absence of the various products, ND = not detectable. | | |

After engagement of CD23 by IgE-containing immune complexes, the production of a large number of mediators and cytokines (especially TNF) and of products derived from the oxidative metabolism, such as the nitrogenous derivatives, is induced. This stimulation redefines in vitro an inflammatory reaction of allergic type.

The results obtained are summarized in the following two tables:

**Table 2A:**

| Production of nitro derivatives (NO₂⁻ µM) after stimulation, by IgE-containing immune complexes ± 10 mg/ml H37 | | | | | |
|---|---|---|---|---|---|
| Cells | | Expt-1 | Expt-2 | Expt-3 | Expt-4 |
| Macropbages | | 25(5)* | 30(2) | 55(11) | 15(2) |
| | +H37 | 15(2) | 17(75) | 21(50) | 6(2) |
| Keratinocytes | | 17(13) | 13(3) | 23(12) | 13(5) |
| | +H37 | 12(11) | 8(2) | 10(10) | 8(6) |

| | | | | | |
|---|---|---|---|---|---|
| * The values in brackets are those for cells not stimulated by the IgE-containing immune complexes. | | | | | |

The H37 product exhibits an inhibitory activity on the production of nitro derivatives by the macrophages and the keratinocytes stimulated by the IgE-containing immune complexes.

**Table 2B:**

| Production of TNF (pg/ml) after stimulation by IgE-containing immune complexes ± 10 mg/ml H37 | | | | | |
|---|---|---|---|---|---|
| Cells | | Expt-1 | Expt-2 | Expt-3 | Expt-4 |
| Macrophages | | 975(105)* | 275(35) | 455(40) | 310(89) |
| | +H37 | 850(105) | 215(25) | 365(35) | 275(87) |
| Keratinocytes | | 185(ND) | 158(ND) | 315(35) | 308(ND) |
| | +H37 | 155(ND) | 120(ND) | 245(30) | 276(ND) |

| | | | | | |
|---|---|---|---|---|---|
| * The values in brackets are those for cells not stimulated by the IgE-containing immune complexes, ND = not detectable. | | | | | |

The H37 product slightly decreases the production of TNF by the macrophages and the keratinocytes stimulated by the IgE-containing immune complexes.

It therefore seems that the H37 product has an allergic anti-inflammatory activity. This characteristic is very important because the seriousness of inflammatory responses, especially of allergic origin, results from a disequilibrium of the oxidative metabolism of these cells. It is in particular this disequilibrium which is responsible, at least in part, for regulating the immunological phenomena associated with these reactions: such is the case for specific allergenic reactions and the production of cytokines.

### Example 2: Study of the effect of H37 on allogenic mixed lymphoepidermal cultures (MLEC) (presentation of the antigen to the T lymphocytes)

The principle of mixed lymphoepidermal cultures (MLEC) is that of mixed lymphocyte cultures, the only difference being that the stimulating population is a suspension of complete epidermal cells (keratinocytes, Langerhans' cells and the like). As this coculture makes it possible to evaluate the capacity of the Langerhans' cells to present the antigen to the T lymphocytes, it is, in particular, very useful for testing the molecules (or stress) capable of modifying the cutaneous immune responses.

In short, batches of control complete epidermal cells, as well as batches of epidermal cells, are cultured with T lymphocytes. Treatment with the exogenous molecule is carried out at the time of the coculture. The lymphocyte proliferation induced by the epidermal cells in then measured. This method makes it possible to monitor the immune functions of the Langerhans' cells during a lymphoepidermal coculture and their modification by exogenous treatments.

The studies were carried out in an allogenic situation (different donors for the epidermal cells and the T lymphocytes) with freshly collected complete epidermal cells obtained from mammary plastic surgery.

### Procedure

This study was carried out with epidermal cells obtained from 2 or 3 different donors.

The epidermal cells are cocultured with allogenic T lymphocytes in 96-well plates: the number of complete epidermal cells is equivalent to the number of lymphocytes (10⁵ epidermal cells - 10⁵ T lymphocytes per well).

The total coculture time is 6 days, the incorporation of tritiated thymidine being carried out 18 hours before the end of the coculture (5th day).

Lymphocyte proliferation is determined by measurement of the incorporation of tritiated thymidine (radioactivity count after "mashing" the plates).

The molecules (H37 extract or cyclosporin) were added at the time of the coculture of the epidermal cells and of the allogenic T lymphocytes.
- Samples treated with the test molecules:
   Coculture of the epidermal cells and of the allogenic T lymphocytes with the molecule to be studied in increasing concentrations by addition of a small volume of a concentrated solution of the molecule (4 final concentrations). For H37, which is soluble in water, the stock solution was prepared in water and then in the medium. For cyclosporin, which is soluble in DMSO, the stock solution was prepared in DMSO and then the daughter solutions in the medium.
   Small proportions of final DMSO were used in the wells (0.1% final for the control DMSO and for cyclosporin).
   The negative controls are the following:
- untreated epidermal cells alone (control EC)
- untreated T lymphocytes alone (control PBMC)
   other controls: epidermal cells alone, treated with DMSO (solvent used for cyclosporin), T lymphocytes alone, treated with DMSO (solvent used for cyclosporin), epidermal cells alone, treated with the molecule at the highest concentration and T lymphocytes alone, treated with the molecule at the highest concentration.

The positive controls are the following:
- epidermal cells + allogenic T lymphocytes (EC = control PBMC)
- epidermal cells + T lymphocytes treated with 0.1% (v/v) DMSO (solvent used for cyclosporin)

The standard is cyclosporin (Cyclo) 10⁶ M.

The product H37 was used at the following final concentrations:
1: 0.03%
2: 0.01%
3: 0.001%
4: 0.0001%

### Results

Preliminary experiments showed that the H37 product, up to the concentration 0.03%, did not show any cytotoxic activity both on the lymphocytes and the epidermal cells.

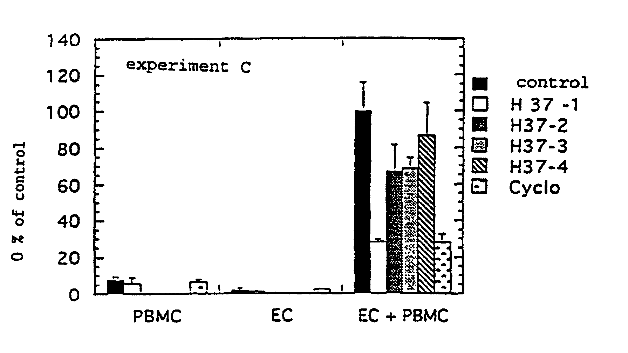

The MLEC results are presented in the figure in the annex.

The following symbols were used:
- **PBMC :**: T lymphocytes alone
- **EC :**: complete epidermal cells alone
- Allogenic **PBMC + EC** :: complete epidermal cells in the presence of T lymphocytes

The results are presented as percentage incorporation of tritiated thymidine (%). The 100% value corresponds to the level of incorporation of thymidine for the EC+PBMC system (complete epidermal cells in the presence of allogenic blood T lymphocytes) in the absence of any product (control).

The experiments represented show that the proliferation of the T lymphocytes occurs in the presence of complete epidermal cells (increase in the incorporation of tritiated thymidine). The amplification of lymphocyte proliferation is linked to the strain of epidermal cells which is different in each experiment. This induced proliferation corresponds to the presentation, by the Langerhans' cells of the preparation of complete epidermal cells, of the antigen to the T lymphocytes.

Cyclosporin, which is a known immunomodulator, inhibits lymphocyte proliferation in MLEC (70% inhibition).

The product H37 has a marked inhibitory effect on lymphocyte proliferation in the MLEC at the concentrations 1 (0.03%) and 2 (0.01%).

These results show that the product H37 has immunomodulatory properties demonstrated during MLEC "in vitro".

## Claims

1. Use of at least one active principle, capable of being obtained by extraction from Ginkgo biloba, for the preparation of a composition with immunomodulatory activity via the topical route.

2. Use of at least one active principle capable of being obtained from Ginkgo biloba according to Claim 1, **characterized in that** the composition is intended to prevent or to treat immunodependent skin reactions.

3. Use according to either of Claims 1 and 2, **characterized in that** the active principle(s) are present in the composition in the form of an extract obtained from Ginkgo biloba leaves.

4. Use according to Claim 3, **characterized in that** the Ginkgo biloba extract has a terpene concentration of less than 3% w/w of dry matter.

5. Use according to either of Claims 3 and 4, **characterized in that** the Ginkgo biloba extract has a concentration of flavone heterosides greater than 24% w/w of dry matter.

6. Use according to one of Claims 1 to 4, **characterized in that** the composition is intended for the treatment or for the prevention of intolerance and/or allergy reactions of the skin and of the mucous membranes.

7. Use according to one of Claims 1 to 6, **characterized in that** the composition is intended for the treatment or prevention of a condition chosen from the group consisting of: atopy, vitiligo, psoriasis, erythema multiforme, neurodermatitis, xeroderma, urticaria, pemphigus, rosacea, lupus erythematosus, dermatitis, benign aestival photodermatitis, eczema and AIDS-related dermatological manifestations.

8. Use according to Claim 7, **characterized in that** the Ginkgo biloba extract is present in the composition at a concentration of between 0.0001 and 10% w/w.

9. Use according to one of Claims 3 to 8, **characterized in that** the Ginkgo extract is present in the composition at a concentration of between 0.01% and 2% w/w.

## Patentansprüche

1. Verwendung mindestens eines aktiven Wirkstoffs, der durch Extraktion aus Ginkgo biloba erhalten werden kann, für die Herstellung einer Zusammensetzung mit immunmodulatorischer Wirkung auf dem topischen Weg.

2. Verwendung mindestens eines aktiven Wirkstoffs, der aus Ginkgo biloba erhalten werden kann, nach Anspruch 1, **dadurch gekennzeichnet, dass** die Zusammensetzung zur Verhütung oder Behandlung immunabhängiger Hautreaktionen gedacht ist.

3. Verwendung nach Anspruch 1 und 2, **dadurch gekennzeichnet, dass** der bzw. die aktive(n) Wirkstoff(e) in der Zusammensetzung in Form eines Extrakts vorliegen, der aus Ginkgo biloba-Blättern erhalten wird.

4. Verwendung nach Anspruch 3, **dadurch gekennzeichnet, dass** der Ginkgo biloba-Extrakt eine Terpen-Konzentration von weniger als 3 % Gew./Gew. an Trockensubstanz aufweist.

5. Verwendung nach Anspruch 3 oder 4, **dadurch gekennzeichnet, dass** der Ginkgo biloba-Extrakt eine Konzentration an Flavonheterosiden von mehr als 24 % Gew./Gew. an Trockensubstanz aufweist.

6. Verwendung nach einem der Ansprüche 1 bis 4, **dadurch gekennzeichnet, dass** die Zusammensetzung für die Behandlung oder die Verhütung von Unverträglichkeits- und/oder allergischen Reaktionen der Haut und der Schleimhautmembranen gedacht ist.

7. Verwendung nach einem der Ansprüche 1 bis 6, **dadurch gekennzeichnet, dass** die Zusammensetzung für die Behandlung oder Verhütung eines Zustands gedacht ist, der ausgewählt ist aus der Gruppe bestehend aus: Atopie, Vitiligo, Psoriasis, Erythema multiforme, Neurodermitis, Xerodermie, Urticaria, Pemphigus, Rosacea, Lupus erythematodes, Dermatitis, gutartiger Sommer-Photodermatitis, Ekzem und mit AIDS in Beziehung stehenden dermatologischen Manifestationen.

8. Verwendung nach Anspruch 7, **dadurch gekennzeichnet, dass** der Ginkgo biloba-Extrakt in der Zusammensetzung bei einer Konzentration zwischen 0,0001 und 10 % Gew./Gew. vorliegt.

9. Verwendung nach einem der Ansprüche 3 bis 8, **dadurch gekennzeichnet, dass** der Ginkgo-Extrakt in der Zusammensetzung bei einer Konzentration zwischen 0,01 % und 2 % Gew./Gew. vorliegt.

## Revendications

1. Utilisation d'au moins un principe actif susceptible d'être obtenu par extraction à partir de Ginkgo biloba pour la préparation d'une composition à activité immunomodulatrice par voie topique.

2. Utilisation d'au moins un principe actif susceptible d'être obtenu à partir de Ginkgo biloba selon la revendication 1, **caractérisée en ce que** la composition est destinée à prévenir ou à traiter les réactions cutanées immunodépendantes.

3. Utilisation selon l'une des revendications 1 et 2, **caractérisée en ce que** le ou les principes actifs sont présents dans la composition sous forme d'un extrait obtenu à partir de feuilles de Ginkgo biloba.

4. Utilisation selon la revendication 3, **caractérisé en ce que** l'extrait de Ginkgo biloba présente une concentration en terpène inférieure à 3% p/p de matière sèche.

5. Utilisation selon l'une des revendication 3 et 4, **caractérisée en ce que** l'extrait de Ginkgo biloba présente une concentration en flavones hétérosides supérieure à 24% p/p de matière sèche.

6. Utilisation selon l'une des revendications 1 à 4, **caractérisée en ce que** la composition est destinée au traitement ou à la prévention des réactions d'intolérance et/ou d'allergie de la peau et des muqueuses.

7. Utilisation selon l'une des revendications 1 à 6, **caractérisée en ce que** la composition est destinée au traitement ou à la prévention d'une affection choisie dans le groupe constituée de : atopie, vitiligo, psoriasis, érythème polymorphe, neurodermite, xérodermie, urticaire, pemphigus, rosacea, lupus érythémateux, dermatite, lucite estivale bénigne, eczéma et manifestations dermatologiques associées au SIDA.

8. Utilisation selon la revendication 7, **caractérisée en ce que** l'extrait de Ginkgo biloba est présent dans la composition à une concentration comprise entre 0,0001 et 10% p/p.

9. Utilisation selon l'une des revendications 3 à 8, **caractérisée en ce que** l'extrait de Ginkgo est présent dans la composition à une concentration comprise entre 0,01% et 2% p/p.
